# EUROPEAN PATENT APPLICATION

(11) **EP 1 952 750 A1**
(43) Date of publication of application: **06.08.2008**
(21) Application number: 06812087.2
(22) Date of filing: 20.10.2006
(51) Int. Cl.: A61B 1/00

(54) **ENDOSCOPE**

(30) Priority: 22.11.2005 JP 2005337235
(71) Applicant: OLYMPUS MEDICAL SYSTEMS CORP., Shibuya-ku Tokyo 151-0072 (JP)
(72) Inventor: UENO, Haruhiko, 2-3 Kuboyama-cho Hachioji-shi Tokyo 192-8512 (JP)
(74) Representative: von Hellfeld, Axel
(86) International application number: PCT/JP2006/320958
(87) International publication number: WO 2007/060800

(57) **Abstract**

An endoscope (12) includes an elongated insertion section (22) inserted into a body cavity and a hard base section (24) provided on a proximal end side of the insertion section (22). Transmission lines, e.g., a light guide fiber (52b, 52c), a CCD cable (54c, 54d), and others are inserted from the inside of the insertion section toward the base section (24). A holding portion (120) which holds the transmission lines while restricting movement of the insertion section (22) and the base section (24) in a direction perpendicular to an axial direction is disposed in the base section (24). A connector portion (28) which connects end portions of the extended transmission lines through the holding portion with an external device is provided to the base section (24).

## Description

### Technical Field

The present invention relates to an endoscope which includes a transmission line through which signals or light is transmitted, the transmission line being arranged inside a base section on a proximal end side of an insertion section.

### Background Art

Jpn. Pat. Appln. KOKAI Publication No. H2(1990)-159243 discloses an endoscope having a frame that prevents noise from being introduced into a cable from an ultrasonic motor acting as a noise source. The frame of the endoscope also functions to shield the cable from not only the noise from the motor but also a bending operation wire. That is, in a conventional noise introduction prevention mechanism, the cable and the noise source (the ultrasonic motor) are arranged to be simply partitioned by a wall portion.

In the endoscope disclosed in the Jpn. Pat. Appln. KOKAI Publication No. H2(1990)-159243, the frame is provided as the wall portion which partitions and shields the ultrasonic motor and the cable, but the cable can be freely moved in a direction perpendicular to an axial direction in the frame. Since the cable can be freely moved in this manner, the cable can be also moved to an undesired position. Therefore, the cable tends to come into contact with other structures.

Further, when a shielded space is large even though the cable is shielded from the noise and others, uniformly shielding the space is difficult, and a portion having a high or low degree of shielding may be produced in some cases. When the cable is moved to a position having a low degree of shielding from a position having a high degree of shielding, noise may possibly be introduced. Therefore, a robust shielding structure is required to uniformly shield the entire space from the noise.

Furthermore, when the cable has a length enabling freely changing its position, the cable corresponding to the length is a waste, and transmission efficiency may be reduced because of the length. For example, in the case of a signal line, noise can hence be readily introduced, and the signal line tends to be attenuated. An optical fiber tends to be attenuated.

### Disclosure of Invention

It is an object of the present invention is to provide an endoscope which is hardly affected by other components or noise and can efficiently transmit signals or light.

To achieve the object, an endoscope according to the present invention includes: an elongated insertion section which is inserted into a body cavity from a distal end portion; a hard base section provided at a proximal end portion of the insertion section; a transmission line which is extended from the inside of the insertion section to the base section and through which signals or light is transmitted; a holding portion which is disposed in the base section and holds the transmission line while restricting movement of the insertion section and the base section in a direction perpendicular to an axial direction thereof; and a connector portion which is provided in the base section and connects an end portion of the extended transmission line to an external device through the holding portion.

### BRIEF DESCRIPTION OF THE SEVERAL VIEWS OF THE DRAWING

FIG. 1 is a schematic view showing an endoscope system according to a first embodiment of the present invention;
FIG. 2A is a schematic partial cross-sectional view showing an electric motor-driven bending type endoscope in the endoscope system according to the first embodiment;
FIG. 2B shows a modification of the schematic partial cross-sectional view depicting the electric motor-driven bending type endoscope in the endoscope system according to the first embodiment;
FIG. 3 is a schematic perspective view showing a frame arranged in a base section of the electric motor-driven bending type endoscope in the endoscope system according to the first embodiment;
FIG. 4 is a schematic vertical sectional view showing the inside of the base section of the electric motor-driven bending type endoscope in the endoscope system according to the first embodiment;
FIG. 5 is a schematic view showing the inside of the base section of the electric motor-driven bending type endoscope in the endoscope system according to the first embodiment;
FIG. 6 is a schematic exploded perspective view showing a coupling arranged between a geared motor and a sprocket in the base section of the electric motor-driven bending type endoscope in the endoscope system according to the first embodiment;
FIG. 7 is a schematic partial cross-sectional view showing an electric motor-driven bending type endoscope in an endoscope system according to a second embodiment of the present invention;
FIG. 8 is a schematic view showing an endoscope system according to a third embodiment of the present invention;
FIG. 9 is a schematic partial cross-sectional view showing an electric motor-driven bending type endoscope in the endoscope system according to the third embodiment;
FIG. 10A is a schematic vertical sectional view showing the inside of a base section of the electric motor-driven bending type endoscope in the endoscope system according to the third embodiment;
FIG. 10B is a view of a coupling piece stopper showing a state observed from a direction of an arrow 10B in FIG. 10A;
FIG. 11A is a schematic vertical sectional view showing the inside of a base section of an electric motor-driven bending type endoscope in an endoscope system according to a fourth embodiment of the present invention;
FIG. 11B is a schematic cross-sectional view taken along line 11B-11B in FIG. 11A;
FIG. 11C is a schematic cross-sectional view taken along line 11B-11B in FIG. 11A;
FIG. 12A shows a modification of the schematic cross-sectional view taken along line 11B-11B of the inside of a base section depicted in FIG. 11A of the electric motor-driven bending type endoscope in the endoscope system according to the fourth embodiment; and
FIG. 12B shows a modification of the schematic cross-sectional view taken along line 11B-11B of the inside of the base section depicted in FIG. 11A of the electric motor-driven bending type endoscope in the endoscope system according to the fourth embodiment.

### Best Mode for Carrying Out the Invention

The best modes for carrying out the present invention will now be explained hereinafter with reference to the drawings.

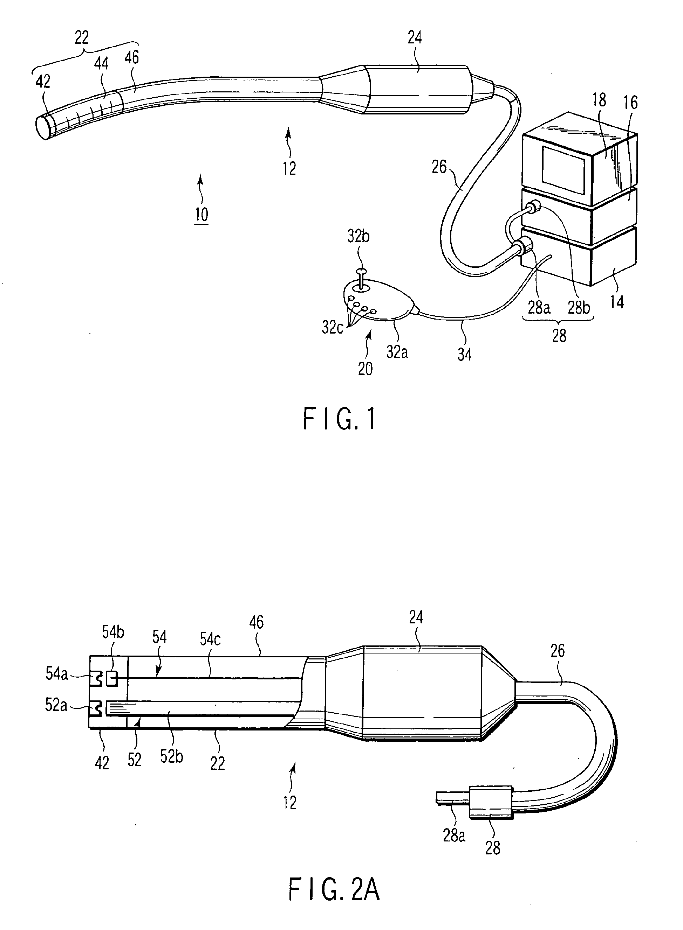

A first embodiment will now be explained with reference to FIGS. 1 to 6.

As shown in FIG. 1, an endoscope system 10 according to the embodiment includes an electric motor-driven bending type endoscope 12, a light source device 14, a processor 16, a monitor 18, and an operating section 20. The light source device 14 transmits light to the endoscope 12. The processor 16 coverts an electric signal from a CCD 54b (see FIG. 2A) provided in a distal end constituting portion 42 of a later-explained insertion section 22 of the endoscope 12 into a picture signal, or controls the CCD 54b. The monitor 18 displays the picture signal processed by the processor 16.

The endoscope 12 includes the elongated insertion section 22, a hard base section 24, and a universal cord (tubular body) 26 having a connector 28 that is connectable with the light source device 14 and the processor 16 at one end portion thereof. A proximal end portion of the insertion section 22 is connected with one end portion (distal end portion) of the base section 24. The other end portion of the universal cord 26 is connected with the other end portion (proximal end portion) of the base section 24. The connector 28 includes a light guide connector 28a to be connected with the light source device 14 and an electrical connector 28b to be connected with the processor 16.

The operating section 20 is provided separately from the endoscope 12. The operating section 20 includes an operating section main body 32a, an operation stick 32b which gives a bending operation instruction, and various kinds of switches 32c. The operating section 20 is electrically connected with the light source device 14 through an operation signal cable 34. Therefore, various kinds of operation instruction signals output when respective operation members, e.g., the operation stick 32b or the switches 32c in the operating section 20 are operated are input to the light source device 14 via the operation signal cable 34. Since the light source device 14 is electrically connected with a later-explained bending drive mechanism 60, the bending drive mechanism 60 is operated by an operation in the operating section 20. Therefore, the operating section 20 can bend a bending portion 44 of an insertion section 22 of the endoscope 12 in an up-and-down direction (UD) direction or a right-and-left (RL) direction.

The insertion section 22 of the endoscope 12 includes a distal end constituting portion 42, the bending portion 44, and a flexible tube portion 46 from a distal end side toward a proximal end side. A proximal end portion of the flexible tube portion 46 is connected with one end portion of the base section 24.

As shown in FIG. 2A, an illumination optical system 52 and an observation optical system 54 are arranged in the endoscope 12. The illumination optical system 52 includes an illumination lens 52a and a light guide fiber 52b. The illumination lens 52a and the light guide fiber 52b are arranged in the distal end constituting portion 42 of the insertion section 22. The light guide fiber 52b is optically connected with a light guide connector 28a through paths of the bending portion 44, the flexible tube portion 46, the base section 24, and the universal cord 26 from the distal end constituting portion 42. Therefore, when illumination light is led to the light guide connector 28a from the light source device 14, the illumination light exits the light guide connector 28a through the light guide fiber 52b and the illumination lens 52a.

The observation optical system 54 includes an object lens 54a, a CCD 54b, and a CCD cable 54c. The object lens 54a and the CCD 54b are arranged in the distal end constituting portion 42 of the insertion section 22. To the CCD 54b is electrically connected the CCD cable 54c through which a signal is transmitted to the CCD 54b when the processor 16 controls the CCD 54b. The CCD cable 54c is electrically connected with the electrical connector 28b (see FIG. 1) from the distal end constituting portion 42 through paths of the bending portion 44, the flexible tube portion 46, the base section 24, and the universal cord 26.

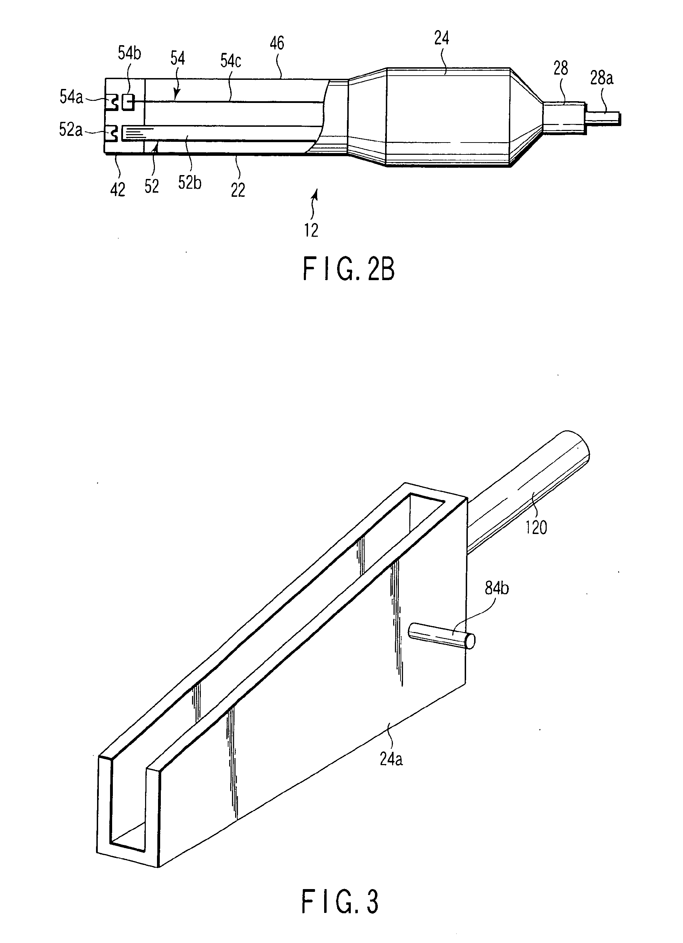

As shown in FIG. 3, a frame (frame body) 24a serving as a base of the base section 24 is arranged in the base section 24. The frame 24a is formed into a box-like shape having substantially trapezoidal side surfaces. Therefore, the frame 24a can be readily formed, and its strength can be easily increased.

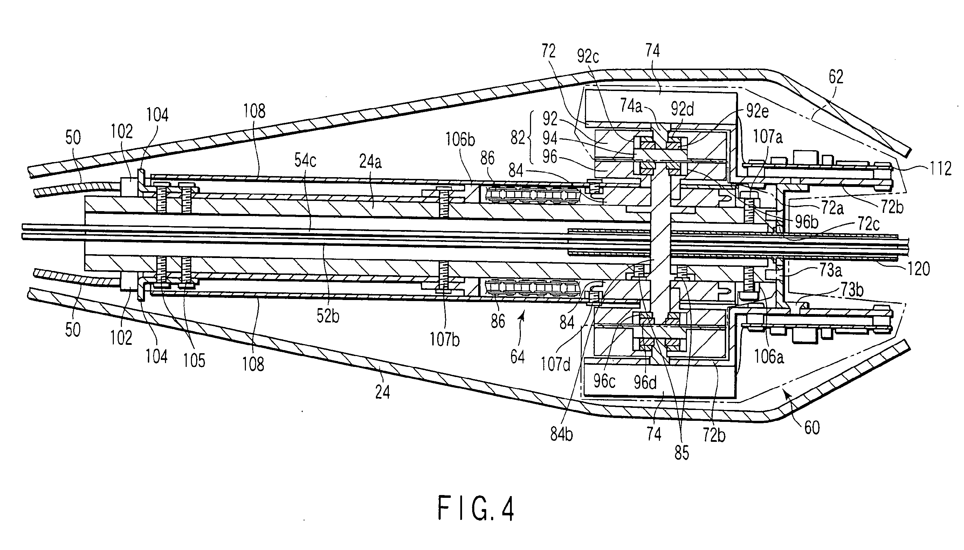

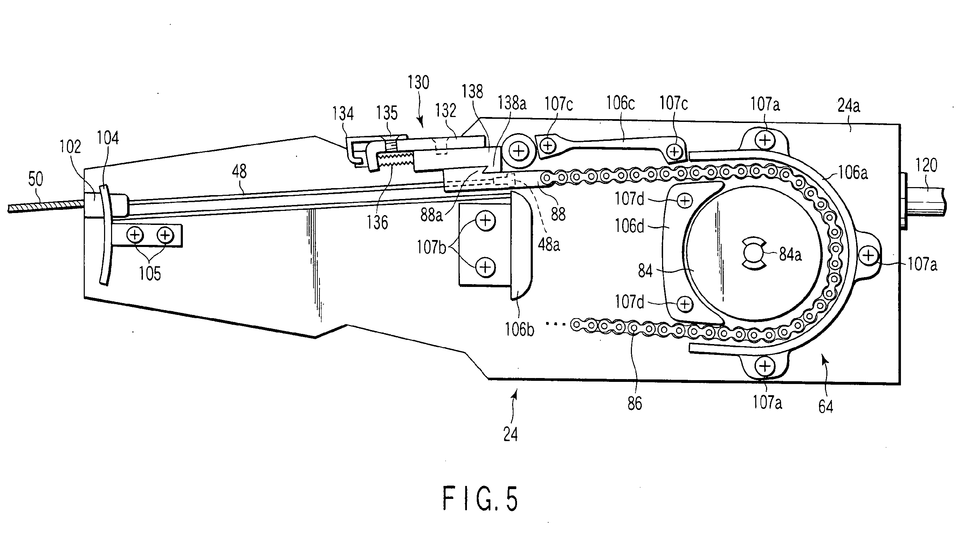

As shown in FIG. 4, the bending drive mechanism 60 which electrically bends the bending portion 44 is arranged in the frame (the frame body) 24a serving as the base of the base section 24. The bending drive mechanism 60 includes a pair of drive sources (motor units) 62 which generate driving forces and a driving force transmission mechanism 64 (see FIG. 5) which independently transmits the driving forces from these drive sources 62 to respective operation wires 48.

The drive sources 62 include first and second motor frames 72a and 72b and a pair of geared motors 74. The first motor frame 72a is fixed to a proximal end portion of the frame (the frame body) 24a of the base section 24 through screws 73a. The second motor frame 72b is fixed to the first motor frame 72a on the outer side of the frame 24a through a screw 73b. The geared motors 74 are fixed to the second motor frame 72b. A drive shaft 74a of the motor 74 is formed with a D-shaped cross section and arranged in a direction perpendicular to a longitudinal direction of the base section 24 to face the inside of the frame 24a.

The driving force transmission mechanism 64 includes a coupling 82, a sprocket 84, a chain 86, and a puller member 88. The coupling 82 is rotated by rotation of the drive shaft 74a of each motor 74. The coupling 82 is also arranged to the sprocket 84. That is, the coupling 82 which transmits a driving force of the drive shaft 74a of the motor 74 to the sprocket 84 is arranged between the geared motor 74 and the sprocket 84. A rotary shaft 84b arranged in an operating portion 84a of the sprocket 84 is fixed by screws 85 to pierce the frame 24a. Therefore, the sprocket 84 is rotated with respect to the rotary shaft 84b with rotation of the coupling 82. It is to be noted that the sprocket 84 for bending in the up-and-down direction and bending in the right-and-left direction is arranged at each end portion of the rotary shaft 84b. Therefore, the rotary shaft 84b is common to each sprocket 84, thereby reducing an axial deviation from the geared motor 74 when assembling each drive source 62.

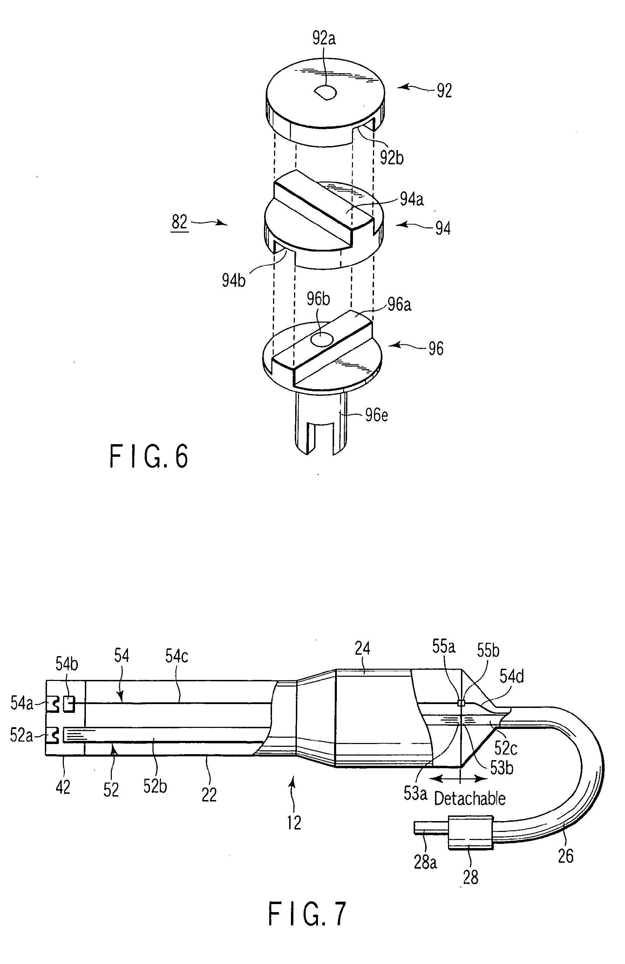

As shown in FIG. 6, the coupling 82 includes first to third members 92, 94, and 96. The first member 92 engages with the second member 94. The second member 94 engages with the third member 96. The second member 94 is arranged between the first and third members 92 and 96.

A D-shaped opening portion 92a in which the D-shaped drive shaft 74a of the motor 74 is fitted and arranged without rotating is formed in one side surface of the first member 92. A concave portion 92b is formed in the other side surface of the first member 92 along a radial direction running through the central axis. A concave portion 92c (see FIG. 4) having a substantially circular cross section is formed at the center of the concave portion 92b. A right screw nut 92d and a left screw nut 92e (see FIG. 4) are arranged in the concave portion 92c. These nuts 92d and 92e are screwed and fixed to the drive shaft 74a of the motor 74.

A convex portion 94a engaging with the concave portion 92b of the first member 92 is formed on one side surface of the second member 94. A concave portion 94b is formed in the other side surface of the second member 94. It is preferable for a longitudinal direction of the concave portion 94b to be perpendicular to that of a longitudinal direction of the convex portion 94a.

A convex portion 96a engaging with the concave portion 94b of the second member 94 is formed on one side surface of the third member 96. A concave portion 96b having a substantially circular cross section is formed in the convex portion 96a. A right screw nut 96c and a left screw nut 96d (see FIG. 4) are arranged in the concave portion 96b. These nuts 96c and 96d are screwed and fixed to the rotary shaft 84b of the sprocket 84. A fork portion 96e which engages with the opening portion 84a (see FIG. 5) of the sprocket 84 and into which the rotary shaft 84b of the sprocket 84 is inserted is formed on the other side surface of the third member 96. That is, the fork portion 96e is opened along the central axis to pierce the axis of the sprocket 84. Therefore, the third member 96 rotates with respect to the fixed rotary shaft 84b, namely, the sprocket 84 engaged with the fork portion 96e of the third member 96 rotates with respect to the rotary shaft 84b.

Therefore, the first to third members 92, 94, and 96, i.e., the coupling 82 integrally rotates with rotation of the drive shaft 74a of the motor 74. Then, the sprocket 84 also rotates with respect to the rotary shaft 84b with rotation of the drive shaft 74a of the motor 74. Even if the drive shaft 74a of the geared motor 74 slightly deviates from the rotary shaft 84b of the sprocket 84 by the coupling 82, the driving force of the motor 74 is smoothly transmitted to the rotary shaft 84b of the sprocket 84.

As shown in FIG. 5, the chain 86 is meshed with outer peripheral teeth of the sprocket 84. The puller member 88 is arranged at an end portion of the chain 86 to pull the operation wire 48. The puller member 88 latches a latch member 48a fixed at a proximal end portion of the operation wire 48. Therefore, the operation wire 48 is coupled with the driving force transmission mechanism 64. Although not shown, a distal end of the operation wire 48 is fixed to the bending portion 44. Therefore, the operation wire 48 is extended to the base section 24 from the bending portion 44 through the inside of a guide tube 50. A coupling piece 102 is fixed to a proximal end portion of the guide tube 50 by, e.g., solder. The coupling piece 102 is latched by a coupling piece stopper 104 fixed to the frame 24a through screws 105. Further, first to third chain guides 106a, 106b, and 106c are respectively fixed to the frame 24a by screws 107a, 107b, and 107c. Furthermore, a fourth chain guide 106d is fixed to a partition plate 108 (see FIG. 4) through screws 107d. Therefore, the chain 86 can smoothly travel, and the chain 86 can be prevented from coming off the sprocket 84.

As shown in FIG. 4, a motor control board (control device) 112 which operates each geared motor 74 is arranged to the second motor frame 72b. The motor control board 112 are electrically connected to the geared motor 74 and the operating section 20. Therefore, a bending signal from the operation stick 32b in the operating section 20 is supplied to the motor control board 112 through the operation signal cable 34, the light source device 14, the connector 28, and the universal cord 26. The motor control board 112 drives each motor 74 based on the bending signal. That is, it controls a rotation amount or a rotating direction of the rotary shaft 84b of the motor 74.

It is to be noted that the rotary shaft 84b of the sprocket 84 is fixed to the frame 24a. Moreover, when the coupling 82 rotates around the rotary shaft 84b, the sprocket 84 rotates. Therefore, when each motor 74, each coupling 82, and each sprocket 84 are provided for the up-and-down (UD) direction and the right-and-left (RL) direction, each sprocket 84 can be independently rotated and operated by control of each motor 74.

A cylindrical holding portion (hollow body) 120 is, e.g., screwed and fixed to the proximal end portion of the frame 24a. At this time, the holding portion 120 is inserted into a through hole 72c of the first motor frame 72a. Therefore, a proximal end portion of the holding portion 120 is extended from the proximal end portion of the frame 24a along the axial direction of the base section 24. The holding portion 120 is formed of an electroconductive material, e.g., aluminum. Alternatively, a thin film formed of an electroconductive material (e.g., aluminum foil) is attached to an inner peripheral surface or an outer peripheral surface of the holding portion 120. Additionally, it is also preferable for the holding portion 120 to have an electroconductive material, e.g., aluminum foil, sandwiched between the outer peripheral surface and the inner peripheral surface thereof. Therefore, the holding portion 120 functions as an electrostatic shield. The CCD cable 54c and the light guide fiber 52b extended from the distal end constituting portion 42 of the insertion section 22 are inserted into the insertion section 22 to be led to the universal cord 26 through the inside of the holding portion 120. An internal diameter of the holding portion 120 is formed to be small so that the CCD cable 54c and the light guide fiber 52b can be prevented from moving in a direction perpendicular to the axial direction. That is, the CCD cable 54c and the light guide fiber 52b are inserted into the holding portion 120 to restrict their positions in the frame 24a. Therefore, the CCD cable 54c and the light guide fiber 52b are inserted into a narrow space and substantially uniformly shielded from noise because the holding portion 120 functions as the electrostatic shield. Accordingly, the holding portion 120 prevents noise from the geared motor 74 and the motor control board 112 from being introduced into the CCD cable 54c.

Meanwhile, a maximum bending angle of the bending portion 44 with respect to the flexible tube portion 46 is determined depending on a type of the endoscope 12. For example, if the endoscope 12 according to the embodiment is used for a large intestine, the maximum bending angle of the bending portion 44 is 180 degrees in the up-and-down (UD) direction and 160 degrees in the right-and-left (RL) direction. The motor control board 112 stores the bending angle, i.e., an angle of rotation (swiveling angle) of the sprocket 84 in a memory (not shown) of the motor control board 112 to control the maximum bending angle.

Here, as shown in FIG. 5, a mechanical stop member 130 is provided in the base section 24 to prevent the bending portion 44 from being broken if a read or write operation of the memory in the motor control board 112 fails for some reason. The stop member 130 includes a stopper pedestal 132, a stopper adjusting screw fixing plate 134, a stopper adjusting screw 136, and a stopper 138. The stopper pedestal 132 is fixed to the frame 24a. The stopper adjusting screw fixing plate 134 is fixed to the stopper pedestal 132 through a screw 135. The stopper 138 includes a protruding portion 138a which comes into contact with a convex portion 88a of the puller member 88 to prevent the puller member 88 from being pulled any further. The stopper adjusting screw 136 adjusts a position of the stopper 138. Further, strength of the stopper 138 is higher than a maximum traction force of each geared motor 74. That is, when the motor 74 is swiveled at a maximum, the stopper 138 overcomes the force to maintain a predetermined state.

A function of the endoscope system 10 according to the embodiment will now be explained. Here, a function of the bending drive mechanism 60 in the base section 24 of the endoscope 12 will be mainly explained.

The operation stick 32b of the operating section 20 is operated in an appropriate direction. There, an operation signal is input to the motor control board 112 of the base section 24 through the operation signal cable 34, the light source device 14, the light guide connector 28a, and the universal cord 26. The motor control board 112 drives the drive shaft 74a of the motor 74 to be rotated based on the input signal.

When the drive shaft 74a of the motor 74 is rotated, the coupling 82 rotates. When the coupling 82 rotates, the sprocket 84 rotates with respect to the rotary shaft 84b. The chain 86 moves based on rotation of the sprocket 84. Therefore, the operation wire 48 moves along its axial direction through the puller member 88 and the latch member 48a. Therefore, the bending portion 44 bends with movement of the operation wire 48.

At this time, when the bending portion 44 is bent, the holding portion 120 allows movement of the CCD cable 54c and the light guide fiber 52b in the axial direction alone but restricts movement of the same in the direction perpendicular to the axial direction. Therefore, the CCD cable 54c and the light guide fiber 52b hardly move. Further, since an inner space of the holding portion 120 having a function as the electrostatic shield is very narrowly formed, a shielded state of the motor 74 against radiated noise can be substantially uniformly maintained in the holding portion 120. Even when the bending portion 44 is bent in this manner, the CCD cable 54c and the light guide fiber 52b hardly move, and the shielded state in the holding portion 120 against the radiated noise can be substantially uniformly maintained, thereby avoiding coming under the influence of the radiated noise as much as possible.

As explained above, according to the embodiment, the following can be said.

The CCD cable 54c and the light guide fiber 52b are inserted into the elongated cylindrical holding portion 120 with the narrow space formed of an electroconductive material, and the CCD cable 54c and the light guide fiber 52b are held in the holding portion 120 in such a manner that these members rarely move. Therefore, the holding portion 120 functions as the electrostatic shield, the shielded state in the holding portion 120 against the radiated noise can be substantially uniformly maintained, and the influence of the radiated noise on the CCD cable 54c and the light guide fiber 52b can be avoided as much as possible.

It is to be noted that the description has been given as to the structure where the connector 28 is provided at the proximal end portion of the hard base section 24 through the universal cord (the tubular body) 26 in this embodiment. Besides, as shown in FIG. 2B, it is also preferable to directly provide the connector 28 at the proximal end portion of the base section 24. Furthermore, the light guide connector 28a is provided in the connector 28 depicted in FIG. 2B, but arranging the electrical connector 28b in the same is also preferable.

A second embodiment will now be explained with reference to FIG. 7. This embodiment is a modification of the first embodiment, and like reference numbers denote like members or members having like functions explained in the first embodiment, thereby omitting a detailed explanation thereof.

In this embodiment, as shown in FIG. 7, a base section 24 and a universal cord 26 are detachably formed.

In this case, a first electric contact 55a is attached to a CCD cable 54c arranged in an insertion section 22 and the base section 24 of an endoscope 12 at a position of a proximal end portion of the base section 24. Moreover, a second electric contact 55b which can be electrically connected with the first electric contact 55a is attached at a position of the other end portion of the universal cord 26. An electric cable 54d is connected with the second electric contact 55b. The electric cable 54d is electrically connected with an electrical connector 28b at one end portion through a path of the universal cord 26.

A first light contact 53a is attached to a light guide fiber 52b at a position of the proximal end portion of the base section 24. Moreover, a second light contact 53b which can be optically connected with the first light contact 53a is attached at a position of the other end portion of the universal cord 26. A light guide fiber 52c is connected with the second light contact 53b. The light guide fiber 52c is optically connected with a light guide connector 28a at one end portion through the path of the universal cord 26.

When attaching the proximal end portion of the base section 24 and the other end portion of the universal cord 26 to each other, they are attached in a state where they are constantly aligned at predetermined positions. When these members are attached in this manner, the first electric contact 55a is electrically connected with the second electric contact 55b, and the first light contact 53a is optically connected with the second light contact 53b.

Other structures are the same as those in the first embodiment, thereby omitting an explanation thereof.

According to this embodiment, when, e.g., carrying the endoscope 12, carriage can be facilitated. Additionally, although not shown in this embodiment, when cleaning a surgical instrument insertion channel and others, cleaning can be readily performed.

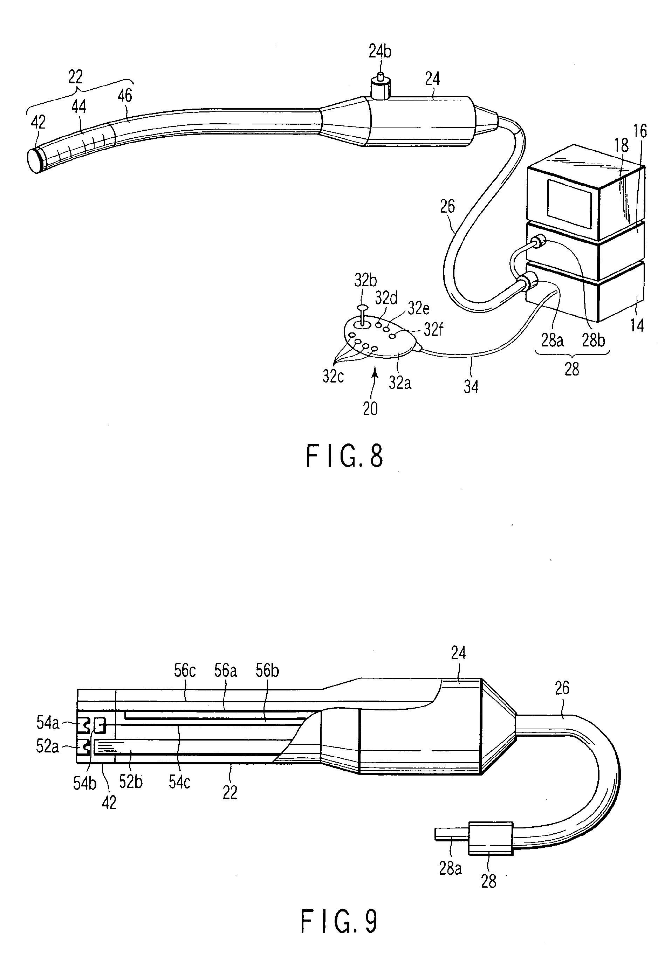

A third embodiment will now be explained with reference to FIGS. 8 to 10B. This embodiment is a modification of the first embodiment, like reference numbers denote like members or members demonstrating like functions explained in the first embodiment, thereby omitting a detailed explanation thereof.

As shown in FIG. 8, an air supply switch 32d, a water supply switch 32e, and a suction switch 32f are further arranged in an operating section main body 32a of an operating section 20. That is, the operating section 20 includes operation buttons which issue an air supply/water supply operation instruction or a suction operation instruction signal besides an operation stick 32b which issues a bending operation instruction. Further, a forceps opening 24b at a proximal end portion of a non-illustrated surgical instrument insertion channel is arranged in a base section 24. Therefore, an elongated surgical instrument can be protruded from a distal end constituting portion 42 from the forceps opening 24b through the base section 24 and an insertion section 22 to perform various kinds of procedures.

As shown in FIG. 9, an air supply duct 56a, a water supply duct 56b, and a suction duct 56c as well as an illumination optical system 52 and an observation optical system 54 are arranged in the insertion section 22, the base section 24, and a universal cord 26 of an endoscope 12. A non-illustrated surgical instrument insertion channel is also arranged in the insertion section 22 and the base section 24 of the endoscope 12. That is, the air supply duct 56a, the water supply duct 56b, the suction duct 56c, and the surgical instrument insertion channel are provided in parallel with in a light guide fiber 52b and a CCD cable 54c.

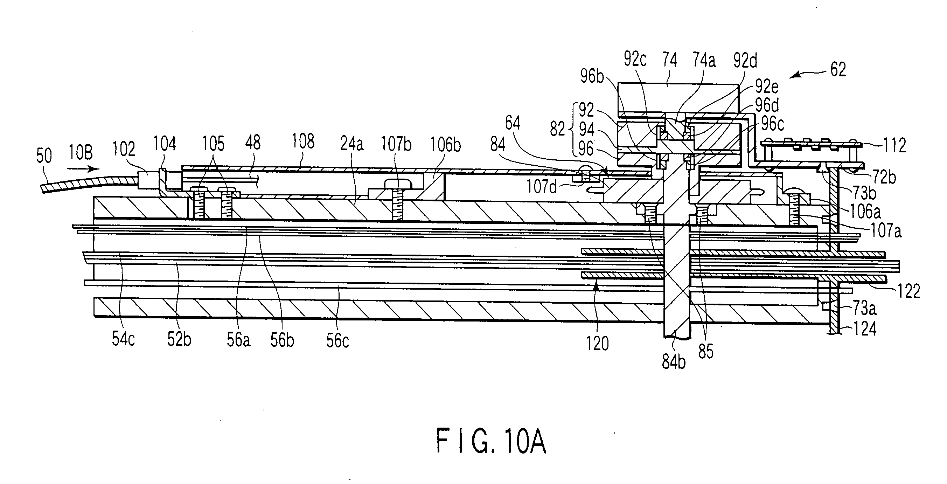

As shown in FIG. 10A, a holding portion 120 integrally includes a cylindrical holding portion main body 122 and an extended portion 124 extended from the main body 122. The extended portion 124 is fixed at a proximal end portion of a frame 24a of the base section 24 through screws 73a.

Like the first embodiment, the light guide fiber 52b and the CCD cable 54c are inserted into the holding portion main body 122. The air supply duct 56a, the water supply duct 56b, and the suction duct 56c are arranged on the outer side of the holding portion main body 122. The air supply duct 56a, the water supply duct 56b, and the suction duct 56c are arranged in a state where they pierce the proximal end portion of the frame 24a.

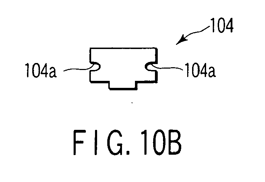

It is to be noted that, as shown in FIG. 10B, a coupling piece stopper 104 includes guide portions 104a through which operation wires 48 are guided. These guide portions 104a maintain a predetermined interval between the operation wires 48 to prevent the wires 48 from entwining.

It is to be noted that this embodiment has the same function and effect as those of the first embodiment, thereby omitting an explanation thereof.

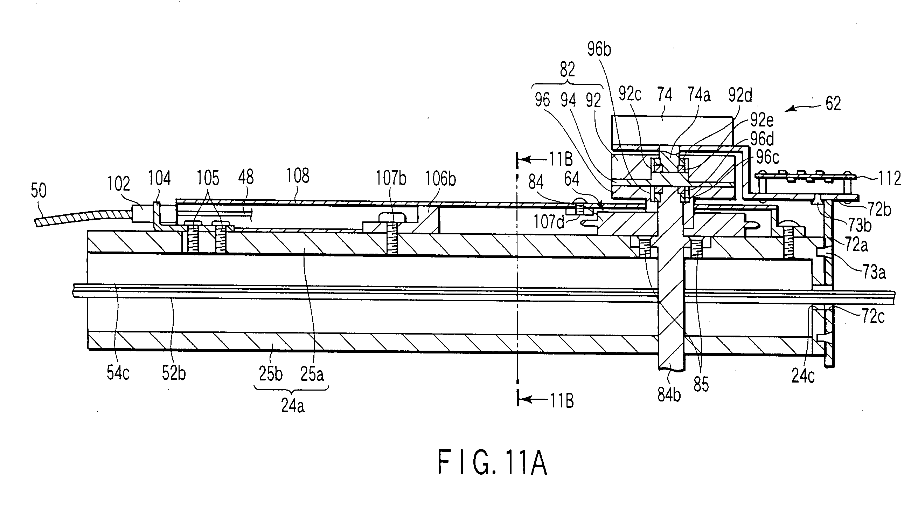

A fourth embodiment will now be explained with reference to FIGS. 11A to 12B. This embodiment is a modification of the first embodiment, and like reference numbers denote like members or members having like functions explained in the first embodiment, thereby omitting a detailed explanation thereof.

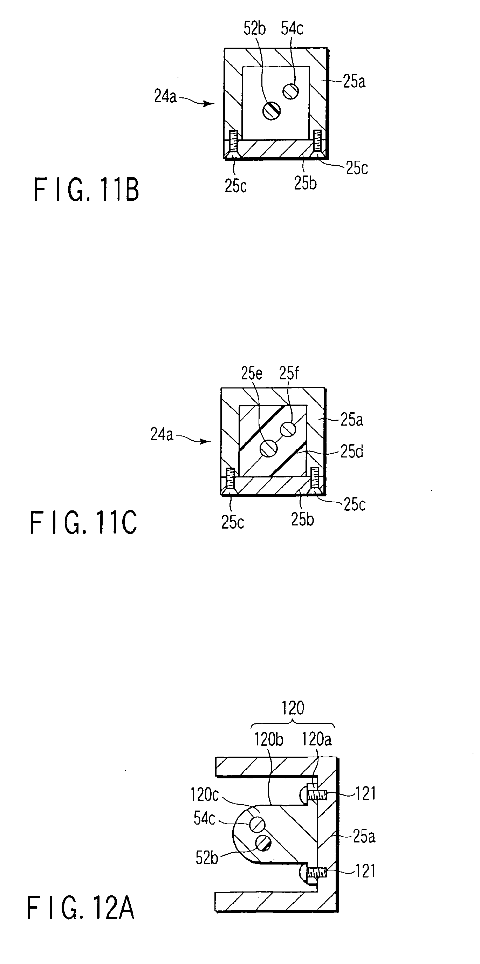

As shown in FIGS. 11A and 11B, a frame 24a includes a U-shaped portion 25a having a substantially U-shaped cross section and a lid portion 25b which closes an opening portion of the U-shaped portion 25a. The lid portion 25b is fixed to the U-shaped portion 25a through screws 25c.

As shown in FIG. 11A, a narrow opening portion 24c is formed at a proximal end portion of the frame 24a to allow movement of a light guide fiber 52b and a CCD cable 54c in an axial direction and prevent movement of the same in a direction perpendicular to the axial direction. An opening portion 72c having substantially the same shape is also formed in a first motor frame 72a coaxially with the opening portion 24c at the proximal end portion of the frame 24a. A thin electroconductive material, e.g., aluminum foil, is attached on inner peripheral surfaces of the U-shaped portion 25a and the lid portion 25b of the frame 24a from these opening portions 24c and 72c near a bending drive mechanism 60. Since the electroconductive material functions as an electrostatic shield and the insides of the opening portions 24c and 72c are narrowly formed, movement in the direction perpendicular to the axial direction is restricted, thereby obtaining a substantially uniform shielded state. Therefore, as explained in the first embodiment, an influence of radiated noise on the CCD cable 54c and the light guide fiber 52b can be avoided as much as possible.

It is to be noted that, as shown in FIG. 11C, arranging a light-weighted position restricting member 25d formed of, e.g., a plastic material in the U-shaped portion 25a of the frame 24a is also preferable. In this case, the position restricting member 25d includes first and second opening portions 25e and 25f. An electroconductive material, e.g., aluminum foil, is attached to inner peripheral surfaces of these first and second opening portions 25e and 25f. Furthermore, although not shown, the light guide fiber 52b is inserted into the first opening portion 25e. Although not shown, the CCD cable 54c is inserted into the second opening portion 25f. These first and second opening portions 25e and 25f are very narrowly formed.

Therefore, since the electroconductive material functions as an electrostatic material and the insides of the opening portions 25e and 25f are narrowly formed, movement in the direction perpendicular to the axial direction is restricted, thereby obtaining a substantially uniformed shielded state. Therefore, as explained in the first embodiment, the influence of the radiated noise on the CCD cable 54c and the light guide fiber 52b can be avoided as much as possible.

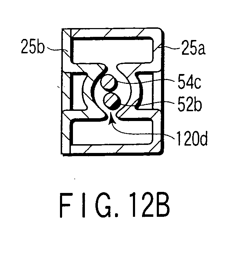

A modification of this embodiment will now be explained with reference to FIGS. 12A and 12B.

As shown in FIG. 12A, a frame 24a is formed with a U-shaped cross section. A holding portion 120 is fixed to a bottom portion of the frame 24a through screws 121. The holding portion 120 integrally includes a flange portion 120a which retains the screws 121 with respect to the frame 24a and an upright portion 120b which is upright from the bottom portion of the frame 24a toward a central portion of the frame 24a. A through hole 120c is formed in the upright portion 120b at a substantially central position of the frame 24a. The holding portion 120 is formed of an electroconductive material, e.g., aluminum. Alternatively, the holding portion 120 is formed of, e.g., a plastic material, and a thin electroconductive material, e.g., aluminum foil, is attached to an inner peripheral surface of the through hole 120c. A light guide fiber 52b and a CCD cable 54c are inserted into the through hole 120c. The through hole 120c is narrowly formed to allow movement of the light guide fiber 52b and the CCD cable 54c in an axial direction but restrict movement of the same in a direction perpendicular to the axial direction.

Therefore, like the above explanation, noise can be prevented from being introduced into the light guide fiber 52b and the CCD cable 54c arranged in the through hole 120c having an electrostatic shielding function.

As shown in FIG. 12B, the frame 24a is deformed at a position near a bending drive mechanism 60. Here, the U-shaped portion 25a and the lid portion 25b are respectively deformed, and a substantially circular concave portion 120d having the light guide fiber 52b and the CCD cable 54c arranged therein is formed at a substantially central position of the frame 24a. The concave portion 120d is narrowly formed to allow movement of the light guide fiber 52b and the CCD cable 54c in the axial direction but restrict movement of the same in the direction perpendicular to the axial direction. Additionally, a space between the U-shaped portion 25a and the lid portion 25b is narrowly formed to prevent the light guide fiber 52b and the CCD cable 54c from outwardly protruding from the concave portion 120d.

Therefore, like the above explanation, noise can be prevented from being introduced into the light guide fiber 52b and the CCD cable 54c arranged in the through hole 120d having the electrostatic shielding function.

Although the several embodiments have been specifically explained with reference to the drawings, the present invention is not restricted to the foregoing embodiments and include all embodiments carried out without departing from the scope of the invention.

### Industrial Applicability

According to the present invention, it is possible to provide an endoscope which is hardly affected by other components or noise and can efficiently transmit signals or light.

## Claims

1. An endoscope (12) **characterized by** comprising:
an elongated insertion section (22) having a distal end portion and a proximal end portion is inserted into a body cavity from the distal end portion;
a hard base section (24) provided at the proximal end portion of the insertion section;
a transmission line (52b, 54c) which is extended from the inside of the insertion section to the base section and through which signals or light is transmitted;
a holding portion (120) which is disposed in the base section and holds the transmission line while restricting movement of the insertion section and the base section in a direction perpendicular to an axial direction thereof; and
a connector portion (28) which is provided in the base section and connects an end portion of the extended transmission line to an external device through the holding portion.

2. The endoscope (12) according to claim 1,
**characterized in that**
a tube body (26) having a path formed therein is arranged between the base section (24) and the connector portion (28), and
the transmission line (52b, 54c) is arranged in the path of the tube body.

3. The endoscope (12) according to claim 1 or 2,
**characterized in that**
the insertion section (22) includes a bendable bending portion (44), and
the base section (24) includes a drive mechanism (60) driven when operating the bending portion to be bent and a frame body (24a) which holds the drive mechanism.

4. The endoscope (12) according to claim 3, **characterized by** comprising an operation wire (48) whose distal end is connected with the bending portion (44) of the insertion section (22) and whose proximal' end is connected with the drive mechanism (60) of the base section (24), and
wherein the drive mechanism includes a drive source (62) which generates a driving force, a transmission mechanism (64) which is connected with the proximal end of the operation wire and transmits the driving force of the drive source to the operation wire, and a control device (112) which operates the drive source.

5. The endoscope (12) according to claim 4,
**characterized in that**
the operation wire (48) includes an up-and-down bending operation wire for an up-and-down direction which bends the bending portion (44) in the up-and-down direction and a right-and-left bending operation wire for a right-and-left direction which bends the bending portion in the right-and-left direction,
the transmission mechanism (64) includes a first transmission mechanism connected with the up-and-down operation wire and a second transmission mechanism connected with the right-and-left bending operation wire, and
the first transmission mechanism and the second transmission mechanism are arranged on the outer side of the holding portion (120).

6. The endoscope (12) according to any one of claims 1 to 5, **characterized in that** the holding portion (120) is formed of an electroconductive material.

7. The endoscope (12) according to any one of claims 1 to 5, **characterized in that** the holding portion (120) has a cylindrical shape and includes a layered electroconductive material in at least a part of a space between an inner peripheral surface and an outer peripheral surface thereof.

8. An endoscope (12) **characterized by** comprising:
an elongated insertion section (22) having a distal end portion and a proximal end portion is inserted into a body cavity from the distal end portion;
a hard base section (24) which is provided at the proximal end portion of the insertion section and has a drive mechanism (60);
a tubular body (26) which is extended from the base section toward the proximal end side, has a connector portion (28) connected with an external device at an extended end portion, and has a path formed therein;
a transmission line (52b, 54c) which is arranged from the inside of the insertion section to the path of the tubular body to be connected with the connector portion and through which signals or light is transmitted; and
a holding portion (120) which is disposed in the base section and holds the transmission line while restricting movement of the insertion section and the base section in a direction perpendicular to an axial direction thereof and avoiding coming under an influence of noise when the noise is produced from the drive mechanism.

9. The endoscope (12) according to claim 8,
**characterized in that**
the insertion section (22) includes a bendable bending portion (44),
the endoscope includes an operation wire (48) which has a distal end connected with the bending portion of the insertion section and a proximal end connected with the drive mechanism (60) of the base section (24), and
the drive mechanism includes a drive source (62) which generates a driving force, a transmission mechanism (64) which is connected with the proximal end of the operation wire and transmits the driving force from the drive source to the operation wire, and a control device (112) which operates the drive source.

10. The endoscope (12) according to claim 9,
**characterized in that**
the operation wire (48) includes an up-and-down bending operation wire for an up-and-down direction which bends the bending portion (44) in the up-and-down direction and a right-and-left bending operation wire for a right-and-left direction which bends the bending portion in the right-and-left direction,
the transmission mechanism (64) includes a first transmission mechanism connected with the up-and-down bending operation wire and a second transmission mechanism connected with the right-and-left bending operation wire, and
the first transmission mechanism and the second transmission mechanism are arranged on the outer side of the holding portion (120).

11. The endoscope (12) according to any one of claims 8 to 10, **characterized in that** the holding portion (120) is formed of an electroconductive material.

12. The endoscope (12) according to any one of claims 8 to 10, **characterized in that** the holding portion (120) has a cylindrical shape and includes a layered electroconductive material in at least a part of a space between an inner peripheral surface and an outer peripheral surface thereof.
